# FASCICULE DE BREVET EUROPEEN

(11) **EP 1 627 625 B1**
(45) Date de publication et mention de la délivrance du brevet: **09.09.2009**
(21) Numéro de dépôt: 05291624.4
(22) Date de dépôt: 29.07.2005
(51) Int. Cl.: A61K 8/41, A61K 8/34, A61K 8/04, A61K 8/89, A61Q 5/12

(54) **Composition cosmétique à base d'un tensioactif cationique, d'une silicone aminée, d'un alcool gras et d'un agent propulseur**
Kosmetische Zusammensetzung auf Basis von einem kationischen Tensid, einem Amin enthaltenden Silikon, einem Fettalkohol und einem Treibmittel
Cosmetic composition based on a cationic surfactant, an aminosilicone, a fatty alcohol and a propellant

(30) Priorité: 11.08.2004 FR 0408824
(43) Date de publication de la demande: 22.02.2006
(73) Titulaire: L'ORÉAL, 75008 Paris (FR)
(72) Inventeur: Lazzeri, Pascale, 92300 Levallois-Perret (FR); Sturla, Jean-Michel, 92100 Boulogne (FR)
(74) Mandataire: Casalonga, Axel

(56) Documents cités:
- EP-A- 1 093 805
- WO-A-01/52800
- WO-A-99/22698
- DE-C1- 19 727 903
- JP-A- 2004 143 093
- US-A1- 2003 068 291
- US-A1- 2004 103 488

## Description

La présente invention est relative à de nouvelles compositions cosmétiques, notamment capillaires, comprenant au moins un tensioactif cationique, au moins une silicone aminée au moins un alcool gras, et au moins un agent propulseur. Elle vise également l'utilisation de ces compositions en cosmétique, et en particulier l'utilisation de ces compositions comme après-shampooing.

Le document DE 197 27 903 décrit des compositions de soins capillaires non rinçées comprenant :
- un alcool gras en C₈ à C₁₈,
- un ester d'acide carboxylique en C₈ à C₁₈ et d'alcool primaire ou secondaire en C₁ à C₆,
- une silicone amino fonctionnalisée,
- en option, au moins un agent conditionnant additionnel tel qu'un polymère cationique, un composé du type ammonium quaternaire, une huile minérale ou végétale.

Le document WO 01/52800 décrit une composition pour le soin des cheveux, comprenant :
- au moins un épaississant non ionique amphiphile associatif dans un milieu cosmétiquement acceptable,
- au moins un agent propulseur,
- en option, au moins un agent de traitement capillaire, constitué d'un composé contenant un groupement cationique et/ou d'au moins un composé siliconé comprenant des groupements hydrophiles.

Le document WO 99/22698 décrit des compositions de fixation capillaire comprenant un polymère fixant non siliconé, une microémulsion d'organopolysiloxane, et un support à base d'eau et/ou de solvants organiques.

Le document JP 2004 143093 décrit une composition pour le conditionnement des cheveux comprenant un condensat de polyol, une huile ayant un point de fusion allant de 30 à 55°C, une silicone aminée, un tensioactif non ionique polyoxyalkyléné, un alcool supérieur saturé monofonctionnel et, de préférence, un tensioactif cationique et/ou un polymère cationique.

Le document US 2003/0068291 propose d'améliorer les propriétés cosmétiques des compositions de conditionnement des cheveux, et propose l'emploi d'une combinaison à base d'une émulsion aqueuse d'un copolymère d'une silicone ayant une viscosité dynamique allant de 10⁶ à 100x10⁶ cP avec au moins un épaississant associatif.

Le document EP 1 093 805 propose également d'améliorer les propriétés cosmétiques des compositions de conditionnement des cheveux, et propose l'emploi d'un copolymère siliconé ayant une viscosité dynamique allant de 10⁶ à 100x10⁶ cP en combinaison avec au moins un tensioactif cationique.

Les après-shampooings classiques sous forme de crème permettent d'obtenir de bonnes propriétés cosmétiques des cheveux, telles que le lissage, le démêlage, la brillance. Toutefois, l'utilisateur a parfois la sensation que ses cheveux sont lourds, peu légers, et qu'ils regraissent facilement, tout particulièrement si ses cheveux sont fins. En outre, le dosage de la crème est parfois difficile, et l'utilisateur consomme beaucoup de produit lors de chaque application.

La Demanderesse vient de découvrir de façon surprenante que des compositions moussantes comprenant au moins un tensioactif cationique, au moins une silicone aminée à groupements ammonium quaternaire, au moins un alcool gras solide ou pâteux à température ambiante, et au moins un agent propulseur, permettaient de remédier aux inconvénients mentionnés ci-dessus.

Ella a en particulier constaté que ces compositions étaient crémeuses et onctueuses, et tenaient très bien dans la main.

Elle a également constaté qu'il n'était pas nécessaire d'utiliser ces compositions en grandes quantités, et que lors de l'application, elles se répartissaient et s'étalaient facilement des racines jusqu'aux pointes, le rinçage étant particulièrement aisé.

Elle a enfin remarqué que ces compositions conféraient de bonnes propriétés cosmétiques aux cheveux, et notamment la brillance, le démêlage, le lissage, que les cheveux étaient légers et regraissaient moins vite, et que les cheveux se mettaient en forme très facilement, et durablement.

La présente invention a donc pour objet une composition cosmétique, notamment capillaire, caractérisée en ce qu'elle comprend, dans un milieu cosmétiquement acceptable, au moins un tensioactif cationique, au moins une silicone aminée à groupements ammonium quaternaire au moins un alcool gras solide ou pâteux à température ambiante, et au moins un agent propulseur.

L'invention a également pour objet un dispositif aérosol comprenant une composition selon l'invention.

Un autre objet de l'invention concerne un procédé cosmétique capillaire mettant en oeuvre les compositions selon l'invention.

L'invention a également pour objet l'utilisation des compositions selon l'invention pour le traitement cosmétique des cheveux et du cuir chevelu.

Un autre objet de l'invention est l'utilisation de la composition pour le lavage et/ou le conditionnement des matières kératiniques telles que les cheveux.

D'autres caractéristiques, aspects, objets et avantages de l'invention apparaîtront encore plus clairement à la lecture de la description et des exemples qui suivent.

Par "milieu cosmétiquement acceptable", on entend un milieu compatible avec toutes les matières kératiniques telles que la peau, les cheveux, les ongles, les cils, les sourcils, les lèvres et toute autre zone du corps et du visage.

Le milieu cosmétiquement acceptable est de préférence constitué par de l'eau ou par un ou plusieurs solvants cosmétiquement acceptable tels que des alcools ou par des mélanges eau-solvant(s). Ces solvants sont de préférence des alcools en C1-C4.

Parmi ces alcools, on peut citer l'éthanol, l'isopropanol, l'éthanol étant particulièrement préféré.

Parmi les tensioactifs cationiques on peut citer en particulier (liste non limitative) : les sels d'amines grasses primaires, secondaires
ou tertiaires, éventuellement polyoxyalkylénées ; les sels d'ammonium quaternaire dont les sels d'alkylpyridinium, les sels d'ammonium d'imidazoline, les sels de diammonium quaternaire, les sels d'ammonium contenant au moins une fonction ester.

A titre de sels d'ammonium quaternaire, on peut notamment citer, par exemple :
- ceux qui présentent la formule générale (V) suivante : 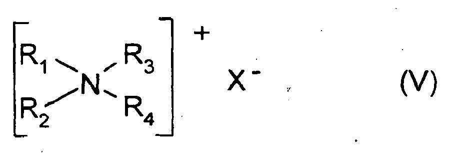
dans laquelle les symboles R1 à R4, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, notamment en C12-C22, alcoxy, polyoxyalkylène (C2-C6), alkylamide, alkyl(C12-C22)amidoalkyle(C2-C6), alkyl(C12-C22)acétate, hydroxyalkyle, comportant environ de 1 à 30 atomes de carbone ; X- est un anion choisi dans le groupe des halogénures, phosphates, acétates, lactates, alkyl(C2-C6)sulfates, alkyl- ou alkylaryl-sulfonates ;
- les sels d'ammonium quaternaire de l'imidazoline comme, par exemple, ceux de formule (VI) suivante : 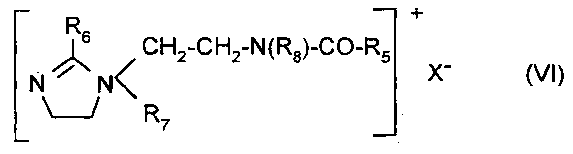
dans laquelle R5 représente un radical alcényle ou alkyle comportant de 8 à 30 atomes de carbone par exemple dérivés des acides gras du suif ou du coprah, R6 représente un atome d' hydrogène, un radical alkyle en C1-C4 ou un radical alcényle ou alkyle comportant de 8 à 30 atomes de carbone, R7 représente un radical alkyle en C1-C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates. De préférence, R5 et R6 désignent un mélange de radicaux alcényle ou alkyle comportant de 12 à 21 atomes de carbone par exemple dérivés des acides gras du suif, R7 désigne méthyle, R8 désigne hydrogène. Un tel produit est par exemple le Quaternium-27(CTFA 1997) ou le Quaternium-83 (CTFA 1997) commercialisés sous les dénominations "REWOQUAT®" W 75, W90, W75PG, W75HPG par la société WITCO,
- les sels de diammonium quaternaire de formule (VII) : 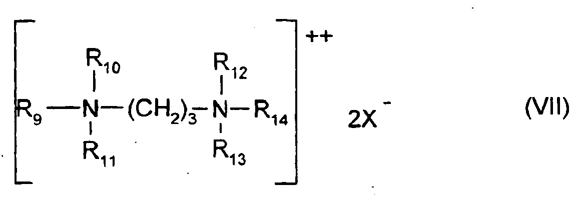
dans laquelle R9 désigne un radical aliphatique comportant environ de 16 à 30 atomes de carbone, R10, R11, R12, R13 et R14, identiques ou différents sont choisis parmi l'hydrogène ou un radical alkyle comportant de 1 à 4 atomes de carbone, et X- est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire contenant au moins une fonction ester, tels que ceux de formule (VIII) suivante : 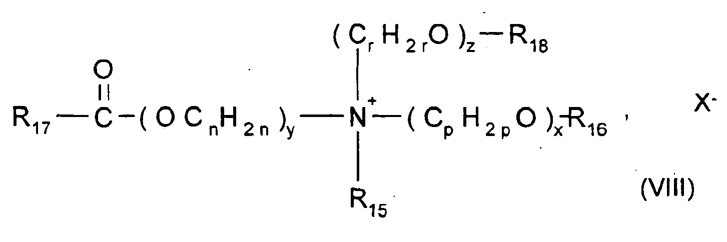
dans laquelle :
R15 est choisi parmi les radicaux alkyles en C1-C6 et les radicaux hydroxyalkyles ou dihydroxyalkyles en C1-C6 ;
R16 est choisi parmi :
   - le radical 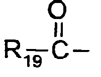
   - les radicaux R20 hydrocarbonés en C1-C22, linéaires ou ramifiés, saturés ou insaturés,
   - l'atome d'hydrogène,
R17 est choisi parmi :
   - le radical 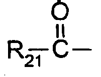
   - les radicaux R22 hydrocarbonés en C1-C6, linéaires ou ramifiés, saturés ou insaturés,
   - l'atome d'hydrogène,
R17, R19 et R21, identiques ou différents, sont choisis parmi les radicaux hydrocarbonés en C7-C21, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10 ;
X- est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

Les radicaux alkyles R15 peuvent être linéaires ou ramifiés, et plus particulièrement linéaires.

De préférence, R15 désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R16 est un radical R20 hydrocarboné, il peut être long et avoir de 12 à 22 atomes de carbone, ou court et avoir de 1 à 3 atomes de carbone.

Lorsque R18 est un radical R22 hydrocarboné, il a de préférence 1 à 3 atomes de carbone.

Avantageusement, R17, R19 et R21, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.

De préférence, x et z, identiques ou différents, valent 0 ou 1.

Avantageusement, y est égal à 1.

De préférence, r, n et p, identiques ou différents, valent 2 ou 3, et encore plus particulièrement sont égaux à 2.

L'anion X- est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement dans la composition selon l'invention, les sels d'ammonium de formule (VIII) dans laquelle :
- R15 désigne un radical méthyle ou éthyle,
- x et y sont égaux à 1 ;
- z est égal à 0 ou 1 ;
- r, n et pt sont égaux à 2 ;
R16 est choisi parmi :
- le radical 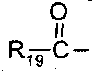
- les radicaux méthyle, éthyle ou hydrocarbonés en C14-C22,
- l'atome d'hydrogène ;
- R18 est choisi parmi :

- le radical 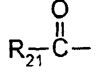
- l'atome d'hydrogène ;
- R17, R19 et R21, identiques ou différents, sont choisis parmi les radicaux hydrocarbonés en C13-C17, linéaires ou ramifiés, saturés ou insaturés, et de préférence parmi les radicaux alkyles et alcényles en C13-C17, linéaires ou ramifiés, saturés ou insaturés.

Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les sels (et notamment les méthosulfates) de dipalmitoyléthylhydroxyéthylméthylammonium, les sels (et notamment les chlorures) de tétraalkylammonium comme, par exemple, les sels (et notamment les chlorures) de dialkyldiméthylammonium ou d'alkyltriméthylammonium dans lesquels le radical alkyle comporte environ de 12 à 22 atomes de carbone, en particulier les sels (et notamment les chlorures) de béhényltriméthylammonium, de distéaryldiméthylammonium, de cétyltriméthylammonium, de benzyldiméthylstéarylammonium ou encore, d'autre part, les sels (et notamment les chlorures) de palmitylamidopropyltriméthylammonium
ou les sels (et notamment les chlorures) de stéaramidopropyldiméthyl-(myristylacétate)-ammonium, et notamment le chlorure de stéaramidopropyldiméthyl-(myristyl acétate)-ammonium commercialisé sous la dénomination CERAPHYL® 70 par la société VAN DYK.

Les tensioactifs cationiques encore plus particulièrement préférés dans la composition de l'invention sont choisis parmi les sels d'ammonium quaternaire, et en particulier parmi le chlorure de béhényltriméthylammonium, le méthosulfate de dipalmitoyléthyl hydroxyéthyl méthyl ammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

Le ou les tensioactifs cationiques peuvent être présents dans la composition à une teneur comprise entre 0,01 et 10%, de préférence entre 0,1 et 5%, encore de préférence entre 0,2 et 4% en poids du poids total de la composition.

Selon l'invention, comme silicone aminée à groupements ammonium quaternaire, on peut citer :
a) les silicones aminées répondant à la formule : 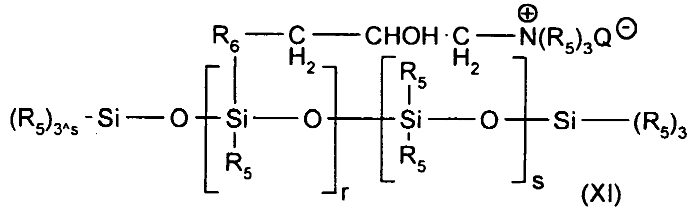 dans laquelle :
   R5 représente un radical hydrocarboné monovalent ayant de 1 à 18 atomes de carbone, et en particulier un radical alkyle en C1-C18, ou alcényle en C2-C18, par exemple méthyle ;
   R6 représente un radical hydrocarboné divalent, notamment un radical alkylène en C1-C18 ou un radical alkylèneoxy divalent en C1-C18, par exemple en C1-C8 relié au Si par une liaison SiC;
   Q- est un anion tel qu'un ion halogénure, notamment chlorure ou un sel d'acide organique (acétate ...);
   r représente une valeur statistique moyenne de 2 à 20 et en particulier de 2 à 8 ;
   s représente une valeur statistique moyenne de 20 à 200 et en particulier de 20 à 50.
   De telles silicones aminées sont décrites plus particulièrement dans le brevet US 4 185 087.
b) les silicones ammonium quaternaire de formule : 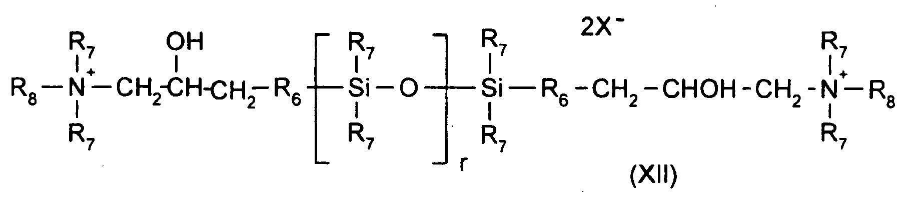 dans laquelle :
   R7, identiques ou différents, représentent un radical hydrocarboné monovalent ayant de 1 à 18 atomes de carbone, et en particulier un radical alkyle en C1-C18, un radical alcényle en C2-C18 ou un cycle comprenant 5 ou 6 atomes de carbone, par exemple méthyle ;
   R6 représente un radical hydrocarboné divalent, notamment un radical alkylène en C1-C18 ou un radical alkylèneoxy divalent en C1-C18, par exemple en C1-C8 relié au Si par une liaison SiC;
   R8, identiques ou différents, représentent un atome d'hydrogène, un radical hydrocarboné monovalent ayant de 1 à 18 atomes de carbone, et en particulier un radical alkyle en C1-C18, un radical alcényle en C2-C18 , un radical -R6-NHCOR7 ;
   X- est un anion tel qu'un ion halogénure, notamment chlorure ou un sel d'acide organique (acétate ...);
   r représente une valeur statistique moyenne de 2 à 200 et en particulier de 5 à 100 ;
   Ces silicones sont par exemple décrites dans la demande EP-A-0530974.

La ou les silicones peuvent être présentes dans la composition à une teneur comprise entre 0,01 et 6%, de préférence entre 0,1 et 3%, encore de préférence entre 0,4 et 2% en poids du poids total de la composition.

L'alcool gras selon l'invention peut linéaire ou ramifié, saturé ou insaturé, et comporter de 8 à 40 atomes de carbone.

L'alcool gras peut présenter la structure R-OH, dans laquelle R désigne un radical saturé ou insaturé, linéaire ou ramifié, comportant de 8 à 40 atomes de carbone et de préférence de 8 à 30 ; R désigne de préférence un groupement alkyle en C12-C24 ou alkényle en C12-C24. R peut être substitué par un ou plusieurs groupements hydroxy.

A titre d'exemple on peut citer l'alcool cétylique, l'alcool stéarylique et leurs mélanges.

L'alcool gras peut représenter un mélange d'alcools gras, ce qui signifie que dans un produit commercial peuvent coexister plusieurs espèces d'alcools gras, sous forme d'un mélange.

L'alcool gras est solide ou pâteux à la température de 20°C. Par « alcool gras solide ou pâteux à 20°C », on entend au sens de la présente invention un alcool gras présentant une viscosité mesurée avec un rhéomètre avec un taux de cisaillement de 1s⁻¹ supérieure ou égale à 1 Pa.s.

Le ou les alcools gras peuvent être présents dans la composition à une teneur comprise entre 0,01 et 10%, de préférence entre 0,1 et 5%, encore de préférence entre 0,2 et 4% en poids du poids total de la composition.

Par agent propulseur au sens de l'invention, on entend tout composé gazeux à la température de 20°C et à la pression atmosphérique et susceptible d'être stocké sous pression sous forme liquide ou gazeuse dans un récipient aérosol.

L'agent propulseur peut être choisi parmi les hydrocarbures volatils, éventuellement halogénés, tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyléther et/ou les hydrocarbures volatils non halogénés.

Le ou les agents propulseurs peuvent être présents dans la composition à une teneur comprise entre 1 et 15%, de préférence entre 2 et 10%, encore de préférence entre 3 et 8% en poids du poids total de la composition.

La composition selon l'invention peut contenir en outre au moins un additif choisi parmi les parfums, les filtres, les tensioactifs non ioniques, anioniques, amphotères ou zwitterioniques, les conservateurs, les protéines, les vitamines, les polymères non ioniques, anioniques, cationiques, amphotères ou zwitterioniques, les huiles minérales, végétales ou synthétiques, les silicones non aminées, les cires végétales, les céramides, et tout autre additif classiquement utilisé dans les compositions cosmétiques, tels que les agents antipelliculaires, les agents anti-chute, les colorants, les pigments, les réducteurs.

Ces additifs sont présents dans la composition à des teneurs avantageusement comprises entre 0,001 et 20% en poids du poids total de la composition. La quantité précise de chaque additif est fonction de sa nature et est déterminée facilement par l'homme du métier, et dépendra de l'application capillaire choisie.

L'homme du métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de traitement cosmétique des matières kératiniques conformes à l'invention se présentent sous forme d'une mousse et peuvent être utilisées en application rincée ou non. Elles sont conditionnées dans un dispositif aérosol. Celui-ci est généralement constitué d'un récipient en métal ou en verre ou en matière plastique, éventuellement recouvert intérieurement d'un vernis, ledit récipient étant fermé hermétiquement et pourvu d'un dispositif de prélèvement permettant la sortie du produit sous forme de mousse. Le dispositif de prélèvement comprend généralement un tube plongeur, une valve et un diffuseur muni d'une buse.

Les compositions conformes à l'invention peuvent être utilisées en tant que compositions de soin de cheveux, de mise en forme des cheveux, shampooings, après-shampooings, compositions de conditionnement des cheveux, telles que des compositions destinées à apporter de la douceur aux cheveux, ou encore des compositions de maquillage des cheveux. De préférence, la composition est un shampooing ou un après-shampooing.

L'invention a également pour objet l'utilisation des compositions selon l'invention pour le traitement cosmétique des cheveux et du cuir chevelu.

Un autre objet de l'invention est l'utilisation de la composition pour le lavage et/ou le conditionnement des matières kératiniques telles que les cheveux.

La présente invention a également pour objet un procédé de traitement cosmétique dans lequel la composition cosmétique selon l'invention est appliquée sur les cheveux, humides ou secs, cette application étant éventuellement suivie d'un rinçage. L'application de la composition s'effectue avantageusement après un shampooing.

Dans une forme de réalisation préférée de l'invention, les compositions selon l'invention sont utilisées comme après-shampooings pour le traitement des cheveux et du cuir chevelu.

Elles sont appliquées, dans ce cas-là, sur des cheveux humides ou secs, dans des quantités efficaces pour les traiter, cette application étant suivie d'un rinçage.

Les exemples suivants sont destinés à illustrer l'invention.

### Exemple 1

On a préparé une composition 1 de référence (crème) et une composition 2 selon l'invention (mousse)

| | Composition 1 | Composition 2 |
|---|---|---|
| Stéarate de glycéryle (1) | 1g | 0,95g |
| Méthosulfate de dipalmitoyléthyl hydroxyéthyl méthyl ammonium/alcool cétéarylique (30/70 en poids) (2) | 4,5g | 4,27g |
| Chlorure de cétyl triméthyl ammonium en solution aqueuse à 25% de matière active (3) | 3,2g | 3,04g |
| Polydiméthylsiloxane à groupement alpha-omega acétate d'ammonium quaternisé à chaîne grasse dans le propylène glycol à 50% (4) | 0,6g | 0,57g |
| Mélange isobutane/propane/butane (5) | - | 5g |
| Eau | Qsp 100g | Qsp 100g |

| | | |
|---|---|---|
| (1) produit commercialisé par la société GOLDSCHMIDT (2) produit commercialisé sous la dénomination DEHYQUART F30 par la société COGNIS (3) produit commercialisé sous la dénomination DEHYQUART AOR par la société COGNIS (4) produit commercialisé sous la dénomination ABIL QUAT 3272 par la société GOLDSCHMIDT (5) produit commercialisé sous la dénomination PROPEL 45 par la société REPSOL | | |

Les compositions 1 et 2 sont appliquées sur les cheveux, cette application étant suivie d'un rinçage.

On observe que les cheveux soumis à l'application de la composition 2 selon l'invention sont plus légers, plus individualisés, sont plus faciles à démêler et à lisser que les cheveux soumis à l'application de la composition 1.

### Exemple 2

On a préparé une composition 1 de référence (crème) et une composition 2 selon l'invention (mousse)

| | Composition 1 | Composition 2 |
|---|---|---|
| Alocol cétylstéarylique (C16/C18 30/70) (6) | 0,5g | 0,47g |
| Stéarate de glycéryle (1) | 1g | 0,95g |
| Méthosulfate de dipalmitoyléthyl hydroxyéthyl méthyl ammonium/alcool cétéarylique (2) | 4,5g | 4,27g |
| Chlorure de cétyl triméthyl ammonium en solution aqueuse (3) | 3,2g | 3,04g |
| Polydiméthylsiloxane à groupement alpha-oméga acétate d'ammonium quaternisé à chaîne grasse dans le propylène glycol à 50% (4) | 0,75g | 0,71g |
| Polydiméthylsiloxane à groupements alpha-oméga acétate d'ammonium quaternaire à chaîne grasse coco en solution à 95% dans le propylène glycol (7) | 0,75g | 0,71g |
| Mélange isobutane/propane/butane | - | 5g |
| Eau | Qsp 100g | Qsp 100g |

| | | |
|---|---|---|
| (6) produit commercialisé sous la dénomination CRODACOL 1618 par la société CRODA (7) produit commercialisé sous la dénomination ABIL QUAT 3474 par la société GOLDSCHMIDT | | |

Les compositions 1 et 2 sont appliquées sur les cheveux, cette application étant suivie d'un rinçage. L'application de la mousse est plus facile que celle de la crème et procure une meilleure répartition de la racine à la pointe.

On observe que les cheveux soumis à l'application de la composition 2 selon l'invention sont plus légers, plus individualisés, sont plus faciles à démêler et à lisser que les cheveux soumis à l'application de la composition 1.

### Exemple 3

On a préparé une composition selon l'invention (mousse)

| | Composition |
|---|---|
| Alcool cétylstéarylique (C16/C18 30/70) (6) | 2,8g |
| Huile de palme | 1g |
| Chlorure de cétyl triméthyl ammonium en solution aqueuse (3) | 3,04g |
| Polydiméthylsiloxane à groupement alpha-oméga acétate d'ammonium quaternisé à chaîne grasse dans le propylène glycol à 50% (4) | 0,57g |
| Mélange isobutane/propane/butane | 5g |
| Eau | Qsp 100g |

La composition est appliquée sur les cheveux, cette application étant suivie d'un rinçage.

On observe que les cheveux sont légers, bien individualisés, et sont faciles à démêler et à lisser.

## Revendications

1. Composition cosmétique, notamment capillaire, **caractérisée en ce qu'**elle comprend, dans un milieu cosmétiquement acceptable, au moins un tensioactif cationique, au moins une silicone aminée à groupements ammonium quaternaire, au moins un alcool gras solide ou pâteux à température ambiante et au moins un agent propulseur.

2. Composition selon la revendication 1, **caractérisée en ce que** le tensioactif cationique est choisi parmi les sels d'amines grasses primaires, secondaires ou tertiaires, éventuellement polyoxyalkylénées ; les sels d'ammonium quaternaire.

3. Composition selon la revendication 2, **caractérisée en ce que** les sels d'ammonium quaternaire sont choisis parmi :
- ceux qui présentent la formule générale (V) suivante : 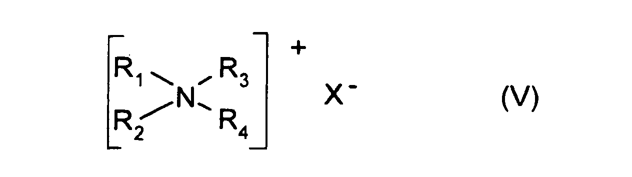 dans laquelle les symboles R1 à R4, qui peuvent être identiques ou différents, représentent un radical aliphatique, linéaire ou ramifié, comportant de 1 à 30 atomes de carbone, ou un radical aromatique tel que aryle ou alkylaryle ; X- est un anion choisi dans le groupe des halogénures, phosphates, acétates, lactates, alkyl(C2-C6)sulfates, alkyl- ou alkylaryl-sulfonates ;
- les sels d'ammonium quaternaire de l'imidazoline de formule (VI) suivante : 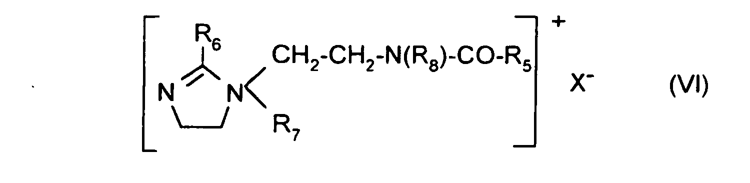 dans laquelle R5 représente un radical alcényle ou alkyle comportant de 8 à 30 atomes de carbone, R6 représente un atome d'hydrogène, un radical alkyle en C1-C4 ou un radical alcényle ou alkyle comportant de 8 à 30 atomes de carbone, R7 représente un radical alkyle en C1-C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates,
- les sels de diammonium quaternaire de formule (VII) : 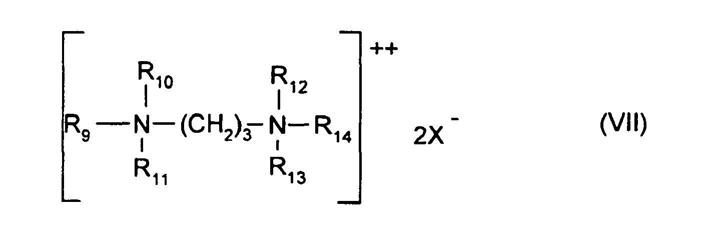 dans laquelle R9 désigne un radical aliphatique comportant de 16 à 30 atomes de carbone, R10, R11, R12, R13 et R14, identiques ou différents sont choisis parmi l'hydrogène ou un radical alkyle comportant de 1 à 4 atomes de carbone, et X- est un anion choisi dans le groupe des halogénures, acétates, phosphates, nitrates, éthylsulfates et méthylsulfates ;
- les sels d'ammonium quaternaire contenant au moins une fonction ester, de formule (VIII) suivante : 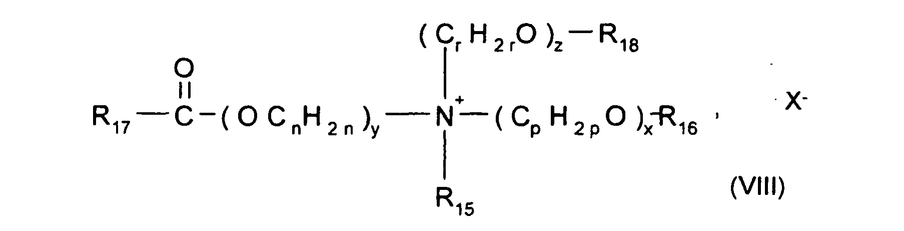 dans laquelle :
R15 est choisi parmi les radicaux alkyles en C1-C6 et les radicaux hydroxyalkyles ou dihydroxyalkyles en C1-C6 ;
R16 est choisi parmi :
- 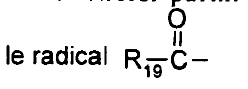
- les radicaux R20 hydrocarbonés en C1-C22, linéaires ou ramifiés, saturés ou insaturés,
- l'atome d'hydrogène,
R17 est choisi parmi :
- 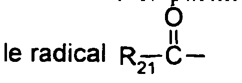
- les radicaux R22 hydrocarbonés en C1-C6, linéaires ou ramifiés, saturés ou insaturés,
- l'atome d'hydrogène,
R17, R19 et R21, identiques ou différents, sont choisis parmi les radicaux hydrocarbonés en C7-C21, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10 ;
X- est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

4. Composition selon la revendication 3, **caractérisée en ce que** le composé de formule (VIII) est choisi parmi les sels de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges.

5. Composition selon la revendication 3, **caractérisée en ce que** le tensioactif de formule (V) est choisi parmi les sels de dipalmitoyléthylhydroxyéthylméthylammonium, les sels de béhényltriméthylammonium, de distéaryldiméthylammonium, de cétyltriméthylammonium, de benzyldiméthylstéarylammonium, les sels de palmitylamidopropyltriméthylammonium, les sels de stéaramidopropyldiméthyl-(myristylacétate)-ammonium.

6. Composition selon la revendication 1, **caractérisée en ce que** le tensioactif cationique est choisi parmi le chlorure de béhényltriméthylammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

7. Composition selon l'une quelconque des revendications précédentes, **caractérisée en ce que** le tensioactif cationique est présent dans la composition à une teneur comprise entre 0,01 et 10%, de préférence entre 0,1 et 5%, et encore de préférence entre 0,2 et 4% en poids du poids total de la composition.

8. Composition selon l'une quelconque des revendications précédentes, **caractérisée en ce que** la silicone aminée est choisie parmi :
a) les silicones aminées répondant à la formule : 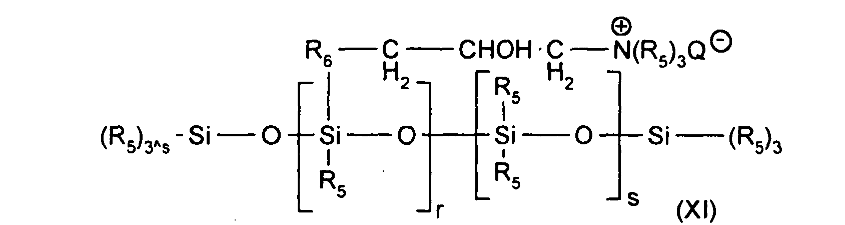 dans laquelle :
R5 représente un radical hydrocarboné monovalent ayant de 1 à 18 atomes de carbone, et en particulier un radical alkyle en C1-C18, ou alcényle en C2-C18 ;
R6 représente un radical hydrocarboné divalent, notamment un radical alkylène en C1-C18 ou un radical alkylèneoxy divalent en C1-C18 ;
Q- est un anion tel qu'un ion halogénure ou un sel d'acide organique;
r représente une valeur statistique moyenne de 2 à 20 et en particulier de 2 à 8 ;
s représente une valeur statistique moyenne de 20 à 200 et en particulier de 20 à 50.
b) les silicones ammonium quaternaire de formule : 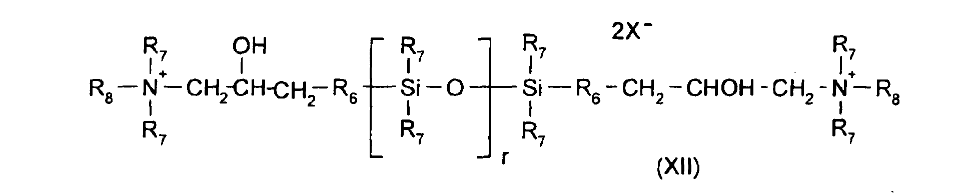
dans laquelle :
R7, identiques ou différents, représentent un radical hydrocarboné monovalent ayant de 1 à 18 atomes de carbone, et en particulier un radical alkyle en C1-C18, un radical alcényle en C2-C18 ou un cycle comprenant 5 ou 6 atomes de carbone ;
R6 représente un radical hydrocarboné divalent, notamment un radical alkylène en C1-C18 ou un radical alkylèneoxy divalent en C1-C18;
R8, identiques ou différents, représentent un atome d'hydrogène, un radical hydrocarboné monovalent ayant de 1 à 18 atomes de carbone, et en particulier un radical alkyle en C1-C18, un radical alcényle en C2-C18 , un radical -R6-NHCOR7 ;
X- est un anion tel qu'un ion halogénure ou un sel d'acide organique;
r représente une valeur statistique moyenne de 2 à 200 et en particulier de 5 à 100 ;

9. Composition selon l'une quelconque des revendications précédentes, **caractérisée en ce que** la silicone aminée est présente dans la composition à une teneur comprise entre 0,01 et 6%, de préférence entre 0,1 et 3%, et encore de préférence entre 0,4 et 2% en poids du poids total de la composition.

10. Composition selon l'une quelconque des revendications précédentes, **caractérisée en ce que** l'alcool gras est linéaire ou ramifié, saturé ou insaturé.

11. Composition selon la revendication 10, **caractérisé en ce que** l'alcool gras présente la structure R-OH, dans laquelle R désigne un radical saturé ou insaturé, linéaire ou ramifié, comportant de 8 à 40 atomes de carbone et de préférence de 8 à 30.

12. Composition selon la revendication 10, **caractérisé en ce que** l'alcool gras est choisi parmi l'alcool cétylique, l'alcool stéarylique, et leurs mélanges.

13. Composition selon l'une quelconque des revendications précédentes, **caractérisée en ce que** l'alcool gras est présent dans la composition à une teneur comprise entre 0,01 et 10%, de préférence entre 0,1 et 5%, et encore de préférence entre 0,2 et 4% en poids du poids total de la composition.

14. Composition selon l'une quelconque des revendications précédentes, **caractérisée en ce que** l'agent propulseur est choisi parmi les hydrocarbures volatils, éventuellement halogénés, le gaz carbonique, le protoxyde d'azote, le diméthyléther (DME), l'azote, l'air comprimé et leurs mélanges.

15. Composition selon la revendication 14, **caractérisée en ce que** l'hydrocarbure volatil est choisi parmi le n-butane, le propane, l'isobutane, le pentane, et leurs mélanges.

16. Composition selon la revendication 14, **caractérisée en ce que** l'agent propulseur est le diméthyléther.

17. Composition selon l'une quelconque des revendications précédentes, **caractérisée en ce que** l'agent propulseur est présent dans la composition à une teneur comprise entre 1 et 15%, de préférence entre 2 et 10%, et encore de préférence entre 3 et 8% en poids du poids total de la composition.

18. Composition selon l'une quelconque des revendications précédentes, **caractérisée en ce que** le milieu cosmétiquement acceptable est constitué par de l'eau ou par un ou plusieurs solvants cosmétiquement acceptables ou par des mélanges eau-solvant(s).

19. Composition selon la revendication 18, **caractérisée en ce que** le solvant cosmétiquement acceptable est choisi parmi l'éthanol et l'isopropanol.

20. Composition selon la revendication 19, **caractérisée en ce que** le solvant cosmétiquement acceptable est l'éthanol.

21. Composition selon l'une quelconque des revendications précédentes, **caractérisée en ce qu'**elle contient en outre au moins un additif choisi parmi les parfums, les filtres, les tensioactifs non ioniques, anioniques, amphotères ou zwitterioniques, les conservateurs, les protéines, les vitamines, les polymères non ioniques, anioniques, cationiques, amphotères ou zwitterioniques, les huiles minérales, végétales ou synthétiques, les agents antipelliculaires, les agents anti-chute, les colorants, les pigments, les réducteurs, les silicones non aminées, les cires végétales, les céramides.

22. Dispositif aérosol comprenant une composition selon l'une quelconque des revendications 1 à 21.

23. Utilisation d'une composition selon l'une quelconque des revendications 1 à 21 pour le traitement cosmétique des cheveux et du cuir chevelu.

24. Procédé de traitement cosmétique des cheveux, **caractérisé en ce qu'**on applique sur les cheveux une composition cosmétique selon l'une des revendications 1 à 21.

25. Procédé selon la revendication 24, **caractérisé en ce que** l'application s'effectue après un shampooing.

## Claims

1. Cosmetic composition, especially a hair composition, **characterized in that** it comprises, in a cosmetically acceptable medium, at least one cationic surfactant, at least one amino silicone with quaternary ammonium groups, at least one fatty alcohol that is solid or pasty at room temperature and at least one propellant.

2. Composition according to Claim 1, **characterized in that** the cationic surfactant is chosen from salts of optionally polyoxyalkylenated primary, secondary or tertiary fatty amines; and quaternary ammonium salts.

3. Composition according to Claim 2, **characterized in that** the quaternary ammonium salts are chosen from: - those of general formula (V) below: 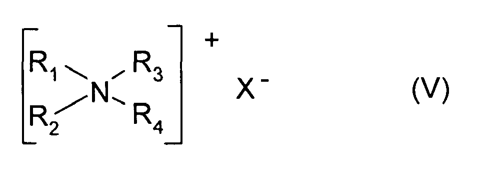 in which the symbols R₁ to R₄, which may be identical or different, represent a linear or branched aliphatic radical containing from 1 to 30 carbon atoms, or an aromatic radical such as aryl or alkylaryl; X- is an anion chosen from the group of halides, phosphates, acetates, lactates, (C₂-C₆) alkyl sulfates and alkyl- or alkylaryl-sulfonates;
- the quaternary ammonium salts of imidazoline of formula (VI) below: 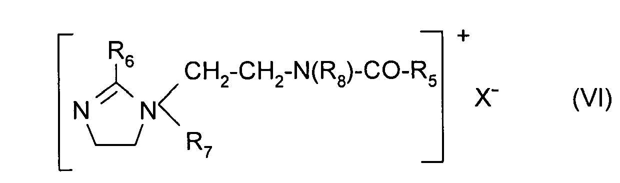 in which R₅ represents an alkenyl or alkyl radical containing from 8 to 30 carbon atoms, R₆ represents a hydrogen atom, a C₁-C₄ alkyl radical or an alkenyl or alkyl radical containing from 8 to 30 carbon atoms, R₇ represents a C₁-C₄ alkyl radical, R₈ represents a hydrogen atom or a C₁-C₄ alkyl radical, and X is an anion chosen from the group of halides, phosphates, acetates, lactates, alkyl sulfates, alkyl sulfonates or alkylaryl sulfonates;
- the diquaternary ammonium salts of formula (VII): 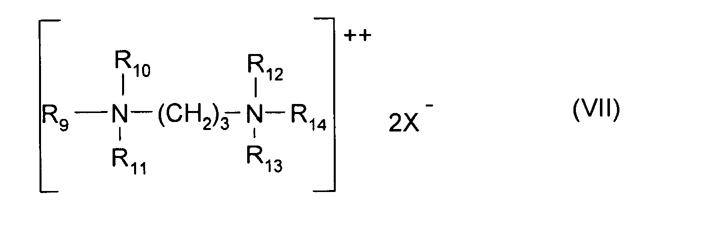 in which R₉ denotes an aliphatic radical containing from about 16 to 30 carbon atoms, R₁₀, R₁₁, R₁₂, R₁₃ and R₁₄, which may be identical or different, are chosen from hydrogen and an alkyl radical containing from 1 to 4 carbon atoms, and X⁻ is an anion chosen from the group of halides, acetates, phosphates, nitrates, ethyl sulfates and methyl sulfates;
- the quaternary ammonium salts containing at least one ester function, of formula (VIII) below: 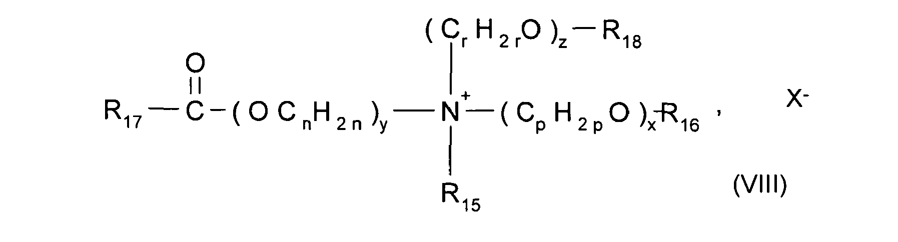 in which:
R₁₅ is chosen from C₁-C₆ alkyl radicals and C₁-C₆ hydroxyalkyl or dihydroxyalkyl radicals;
R₁₆ is chosen from:
- a radical 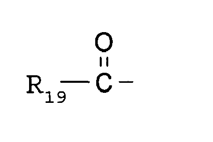
- linear or branched, saturated or unsaturated C₁-C₂₂ hydrocarbon-based radicals R₂₀,
- a hydrogen atom,
R₁₇ is chosen from:
- a radical 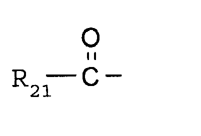
- linear or branched, saturated or unsaturated C₁-C₆ hydrocarbon-based radicals R₂₂,
- a hydrogen atom,
R₁₇, R₁₉ and R₂₁, which may be identical or different, are chosen from linear or branched, saturated or unsaturated C₇-C₂₁ hydrocarbon-based radicals;
r, n and p, which may be identical or different, are integers ranging from 2 to 6;
y is an integer ranging from 1 to 10;
x and z, which may be identical or different, are integers ranging from 0 to 10;
X⁻ is a simple or complex organic or inorganic anion;
with the proviso that the sum x + y + z ranges from 1 to 15, that when x is 0, then R₁₆ denotes R₂₀ and that when z is 0, then R₁₈ denotes R₂₂.

4. Composition according to Claim 3, **characterized in that** the compound of formula (VIII) is chosen from the salts of diacyloxyethyl-dimethylammonium, of diacyloxyethyl-hydroxyethyl-methylammonium, of monoacyloxyethyl-dihydroxyethyl-methylammonium, of triacyloxyethyl-methylammonium, of monoacyloxyethyl-hydroxyethyl-dimethylammonium, and mixtures thereof.

5. Composition according to Claim 3, **characterized in that** the surfactant of formula (V) is chosen from dipalmitoylethylhydroxyethylmethylammonium salts, behenyltrimethylammonium, distearyldimethylammonium, cetyltrimethylammonium and benzyldimethyl-stearylammonium salts, palmitylamidopropyltrimethyl-ammonium salts and stearamidopropyldimethyl(myristyl acetate)ammonium salts.

6. Composition according to Claim 1, **characterized in that** the cationic surfactant is chosen from behenyltrimethylammonium chloride, cetyltrimethylammonium chloride, quaternium-83, behenylamidopropyl 2,3-dihydroxypropyl dimethyl ammonium chloride and palmitoylamidopropyltrimethylammonium chloride.

7. Composition according to any one of the preceding claims, **characterized in that** the cationic surfactant is present in the composition in a content of between 0.01% and 10%, preferably between 0.1% and 5% and more preferably between 0.2% and 4% by weight relative to the total weight of the composition.

8. Composition according to any one of the preceding claims, **characterized in that** the amino silicone is chosen from:
a) amino silicones corresponding to the formula: 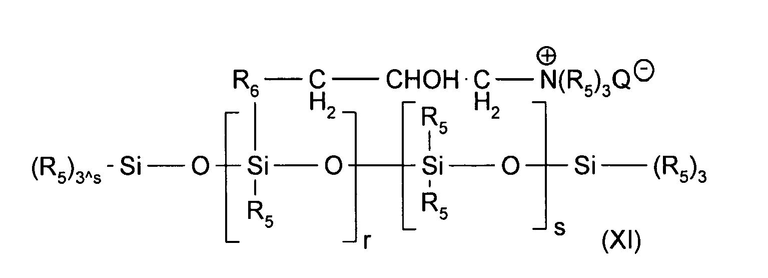 in which:
R₅ represents a monovalent hydrocarbon-based radical containing from 1 to 18 carbon atoms, and in particular a C₁-C₁₈ alkyl or C₂-C₁₈ alkenyl radical;
R₆ represents a divalent hydrocarbon-based radical, in particular a C₁-C₁₈ alkylene radical or a divalent C₁-C₁₈ alkylenoxy radical;
Q⁻ is an anion such as a halide ion or an organic acid salt;
r represents an average statistical value from 2 to 20 and in particular from 2 to 8;
s represents an average statistical value from 20 to 200 and in particular from 20 to 50,
b) quaternary ammonium silicones of formula: 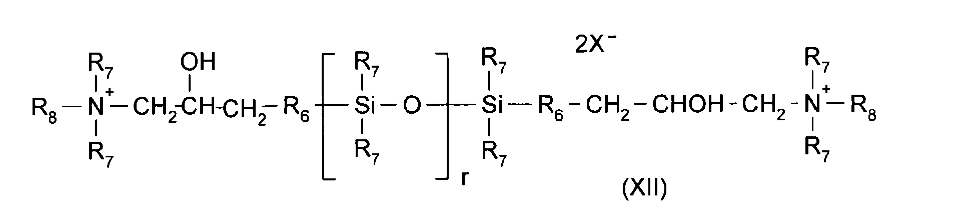
in which:
R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring containing 5 or 6 carbon atoms;
R₆ represents a divalent hydrocarbon-based radical, in particular a C₁-C₁₈ alkylene radical or a divalent C₁-C₁₈ alkylenoxy radical;
R₈, which may be identical or different, represent a hydrogen atom, a monovalent hydrocarbon-based radical containing from 1 to 18 carbon atoms, and in particular a C₁-C₁₈ alkyl radical, a C₂-C₁₈ alkenyl radical or a radical -R₆-NHCOR₇;
X- is an anion such as a halide ion or an organic acid salt;
r represents an average statistical value from 2 to 200 and in particular from 5 to 100.

9. Composition according to any one of the preceding claims, **characterized in that** the amino silicone is present in the composition in a content of between 0.01% and 6%, preferably between 0.1% and 3% and more preferably between 0.4% and 2% by weight relative to the total weight of the composition.

10. Composition according to any one of the preceding claims, **characterized in that** the fatty alcohol is linear or branched, and saturated or unsaturated.

11. Composition according to Claim 10, **characterized in that** the fatty alcohol has the structure R-OH, in which R denotes a saturated or unsaturated, linear or branched radical containing from 8 to 40 and preferably from 8 to 30 carbon atoms.

12. Composition according to Claim 10, **characterized in that** the fatty alcohol is chosen from cetyl alcohol, stearyl alcohol, and mixtures thereof.

13. Composition according to any one of the preceding claims, **characterized in that** the fatty alcohol is present in the composition in a content of between 0.01% and 10%, preferably between 0.1% and 5% and more preferably between 0.2% and 4% by weight relative to the total weight of the composition.

14. Composition according to any one of the preceding claims, **characterized in that** the propellant is chosen from optionally halogenated volatile hydrocarbons, carbon dioxide, nitrous oxide, dimethyl ether (DME), nitrogen and compressed air, and mixtures thereof.

15. Composition according to Claim 14, **characterized in that** the volatile hydrocarbon is chosen from n-butane, propane, isobutane and pentane, and mixtures thereof.

16. Composition according to Claim 14, **characterized in that** the propellant is dimethyl ether.

17. Composition according to any one of the preceding claims, **characterized in that** the propellant is present in the composition in a content of between 1% and 15%, preferably between 2% and 10% and more preferably between 3% and 8% by weight relative to the total weight of the composition.

18. Composition according to any one of the preceding claims, **characterized in that** the cosmetically acceptable medium consists of water or of one or more cosmetically acceptable solvents, or of water-solvent mixtures.

19. Composition according to Claim 18, **characterized in that** the cosmetically acceptable solvent is chosen from ethanol and isopropanol.

20. Composition according to Claim 19, **characterized in that** the cosmetically acceptable solvent is ethanol.

21. Composition according to any one of the preceding claims, **characterized in that** it also contains at least one additive chosen from fragrances, screening agents, nonionic, anionic, amphoteric or zwitterionic surfactants, preserving agents, proteins, vitamins, nonionic, anionic, cationic, amphoteric or zwitterionic polymers, mineral, plant or synthetic oils, antidandruff agents, agents for preventing hair loss, dyes, pigments, reducing agents, non-amino silicones, plant waxes, and ceramides.

22. Aerosol device comprising a composition according to any one of Claims 1 to 21.

23. Use of a composition according to any one of Claims 1 to 21 for the cosmetic treatment of the hair and the scalp.

24. Cosmetic hair treatment process, **characterized in that** a cosmetic composition according to one of Claims 1 to 21 is applied to the hair.

25. Process according to Claim 24, **characterized in that** the composition is applied after shampooing.

## Patentansprüche

1. Kosmetische Zusammensetzung, insbesondere für das Haar, **dadurch gekennzeichnet, daß** sie in einem kosmetisch unbedenklichen Medium mindestens ein kationisches Tensid, mindestens ein Aminosilikon mit quartären Ammoniumgruppen, mindestens einen Fettalkohol, der bei Umgebungstemperatur fest oder pastös ist, und mindestens ein Treibmittel enthält.

2. Zusammensetzung nach Anspruch 1, **dadurch gekennzeichnet, daß** das kationische Tensid unter Salzen von gegebenenfalls polyoxyalkylierten primären, sekundären oder tertiären Fettaminen und quartären Ammoniumsalzen ausgewählt ist.

3. Zusammensetzung nach Anspruch 2, **dadurch gekennzeichnet, daß** die Ammoniumsalze ausgewählt sind unter:
- denjenigen der folgenden allgemeinen Formel (V): 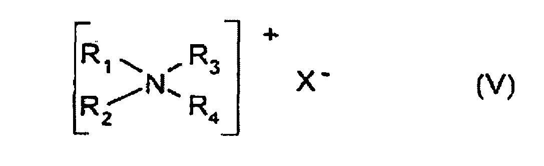 worin die Symbole R1 bis R4 gleich oder verschieden sein können und für einen linearen oder verzweigten aliphatischen Rest mit 1 bis 30 Kohlenstoffatomen oder einen aromatischen Rest wie Aryl oder Alkylaryl stehen und X- ein Anion aus der Gruppe der Halogenide, Phosphate, Acetate, Lactate, (C2-C6)-Alkylsulfate und Alkyl- oder Alkylarylsulfonate ist;
- den quartären Ammoniumsalzen von Imidazolin der folgenden Formel (VI): 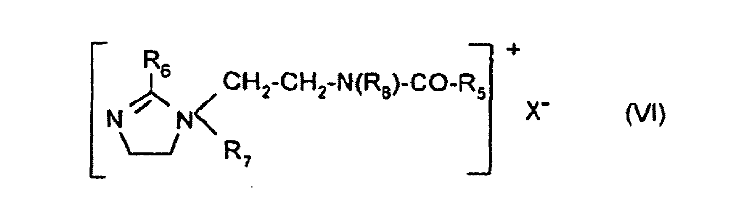 worin R5 für einen Alkenyl- oder Alkylrest mit 8 bis 30 Kohlenstoffatomen steht, R6 für ein Wasserstoffatom, einen C1-C4-Alkylrest oder einen Alkenyl- oder Alkylrest mit 8 bis 30 Kohlenstoffatomen steht, R7 für einen C1-C4-Alkylrest steht, R8 für ein Wasserstoffatom oder einen C1-C4-Alkylrest steht und X ein Anion aus der Gruppe der Halogenide, Phosphate, Acetate, Lactate, Alkylsulfate und Alkyl- oder Alkylarylsulfonate ist;
- den quartären Diammoniumsalzen der Formel (VII): 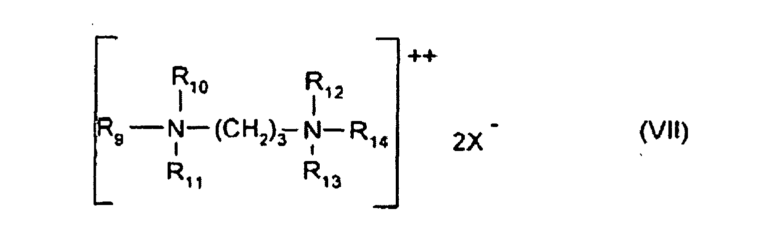 worin R9 für einen aliphatischen Rest mit 16 bis 30 Kohlenstoffatomen steht, R10, R11, R12, R13 und R14 gleich oder verschieden sind und unter Wasserstoff oder einem Alkylrest mit 1 bis 4 Kohlenstoffatomen ausgewählt sind und X- ein Anion aus der Gruppe der Halogenide, Acetate, Phosphate, Nitrate, Ethylsulfate und Methylsulfate ist;
- den quartären Ammoniumsalzen mit mindestens einer Esterfunktion der folgenden Formel (VIII): 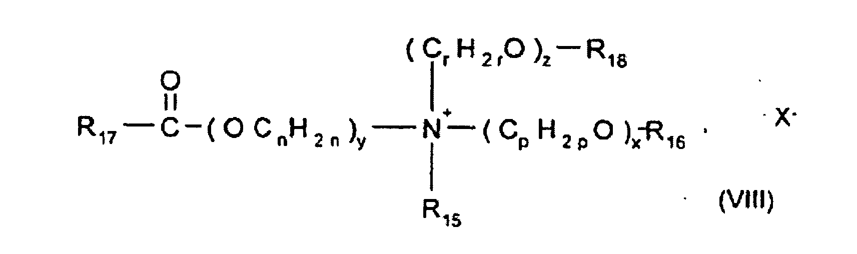 worin:
R15 unter C1-C6-Alkylresten und C1-C6-Hydroxyalkyl- oder -Dihydroxyalkylresten ausgewählt ist; R16 unter:
- einem Rest 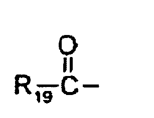
- gesättigten oder ungesättigten, linearen oder verzweigten Cl-C22-Kohlenwasserstoffresten R20 und
- einem Wasserstoffatom ausgewählt ist,
R17 unter:
- einem Rest 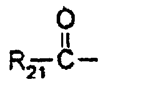
- gesättigten oder ungesättigten, linearen oder verzweigten C1-C6-Kohlenwasserstoffresten R22 und
- einem Wasserstoffatom ausgewählt ist,
R17, R19 und R21 gleich oder verschieden sind und unter gesättigten oder ungesättigten, linearen oder verzweigten C7-C21-Kohlenwasserstoffresten ausgewählt sind;
r, n und p gleich oder verschieden sind und für ganze Zahlen im Bereich von 2 bis 6 stehen;
y für eine ganze Zahl im Bereich von 1 bis 10 steht;
x und z gleich oder verschieden sind und für ganze Zahlen im Bereich von 0 bis 10 stehen;
X- für ein organisches oder anorganisches, einfaches oder komplexes Anion steht;
mit den Maßgaben, daß die Summe x + y + z 1 bis 15 beträgt, R16 im Fall von x gleich 0 für R20 steht und R18 im Fall von z gleich 0 für R22 steht.

4. Zusammensetzung nach Anspruch 3, **dadurch gekennzeichnet, daß** die Verbindung der Formel (VIII) unter Diacyloxyethyl-dimethylammonium-, Diacyloxyethyl-hydroxyethyl-methylammonium-, Monoacyloxyethyl-dihydroxyethyl-methylammonium-, Triacyloxyethyl-methylammonium-, Monoacyloxyethylhydroxyethyl-dimethylammoniumsalzen und Mischungen davon ausgewählt ist.

5. Zusammensetzung nach Anspruch 3, **dadurch gekennzeichnet, daß** das Tensid der Formel (V) unter Dipalmitoylethylhydroxyethylmethylammoniumsalzen, Behenyltrimethylammonium-, Distearyldimethylammonium-, Cetyltrimethylammonium- und Benzyldimethylstearylammoniumsalzen, Palmitylamidopropyltrimethylammoniumsalzen und Stearamido-propyldimethyl(myristylacetat)ammoniumsalzen ausgewählt ist.

6. Zusammensetzung nach Anspruch 1, **dadurch gekennzeichnet, daß** das kationische Tensid unter Behenyltrimethylammoniumchlorid, Cetyltrimethylammoniumchlorid, Quaternium-83, Behenylamidopropyl-2,3-dihydroxypropyl-dimethylammoniumchlorid und Palmitylamidopropyltrimethylammoniumchlorid ausgewählt ist.

7. Zusammensetzung nach einem der vorhergehenden Ansprüche, **dadurch gekennzeichnet, daß** das kationische Tensid in der Zusammensetzung in einer Menge zwischen 0,01 und 10 Gew.-%, vorzugsweise zwischen 0,1 und 5 Gew.-% und weiter bevorzugt zwischen 0,2 und 4 Gew.-%, bezogen auf das Gesamtgewicht der Zusammensetzung, vorliegt.

8. Zusammensetzung nach einem der vorhergehenden Ansprüche, **dadurch gekennzeichnet, daß** das Aminosilikon ausgewählt ist unter:
a) Aminosilikonen der Formel: 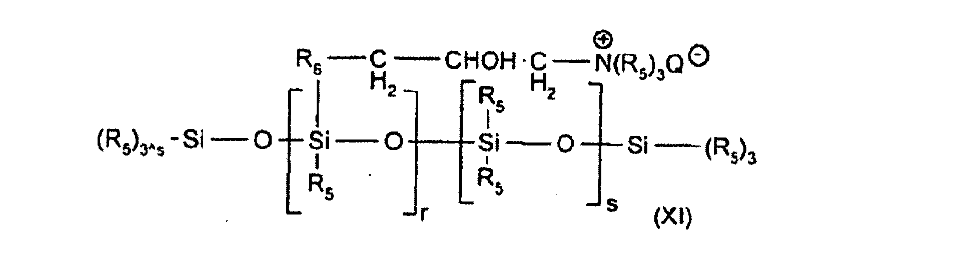 worin:
R5 für einen einwertigen Kohlenwasserstoffrest mit 1 bis 18 Kohlenstoffatomen und insbesondere einen Cl-C18-Alkyl- oder C2-C18-Alkenylrest steht;
R6 für einen zweiwertigen Kohlenwasserstoffrest, insbesondere einen C1-C18-Alkylenrest oder einen zweiwertigen Cl-C18-Alkylenoxyrest steht;
Q- für ein Anion wie ein Halogenidion oder ein Salz einer organischen Säure steht;
r für einen durchschnittlichen statistischen Wert von 2 bis 20 und insbesondere 2 bis 8 steht und
s für einen durchschnittlichen statistischen Wert von 20 bis 200 und insbesondere 20 bis 50 steht; und
b) quartären Ammoniumsilikonen der Formel: 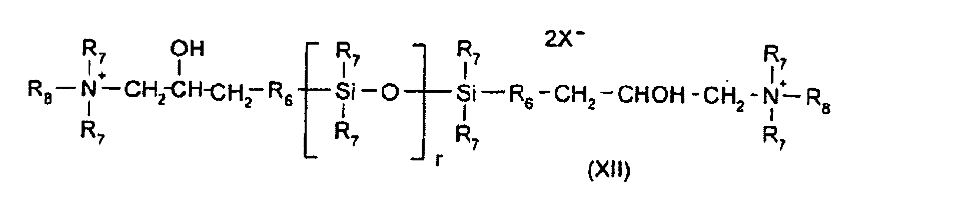
worin:
die Gruppen R7 gleich oder verschieden sind und für einen einwertigen Kohlenwasserstoffrest mit 1 bis 18 Kohlenstoffatomen und insbesondere einen C1-C18-Alkylrest, einen C2-C18-Alkenylrest oder einen Ring mit 5 oder 6 Kohlenstoffatomen stehen;
R6 für einen zweiwertigen Kohlenwasserstoffrest, insbesondere einen C1-C18-Alkylenrest oder einen zweiwertigen C1-C18-Alkylenoxyrest steht;
die Gruppen R8 gleich oder verschieden sind und für ein Wasserstoffatom, einen Kohlenwasserstoffrest mit 1 bis 18 Kohlenstoffatomen und insbesondere einen C1-C18-Alkylrest oder einen C2-C18-Alkenylrest oder einen -R6-NHCOR7-Rest stehen;
X- für ein Anion wie ein Halogenidion oder ein Salz einer organischen Säure steht;
r für einen durchschnittlichen statistischen Wert von 2 bis 200 und insbesondere 5 bis 100 steht.

9. Zusammensetzung nach einem der vorhergehenden Ansprüche, **dadurch gekennzeichnet, daß** das Aminosilikon in der Zusammensetzung in einer Menge zwischen 0,01 und 6 Gew.-%, vorzugsweise zwischen 0,1 und 3 Gew.-% und weiter bevorzugt zwischen 0,4 und 2 Gew.-%, bezogen auf das Gesamtgewicht der Zusammensetzung, vorliegt.

10. Zusammensetzung nach einem der vorhergehenden Ansprüche, **dadurch gekennzeichnet, daß** der Fettalkohol linear oder verzweigt und gesättigt oder ungesättigt ist.

11. Zusammensetzung nach Anspruch 10, **dadurch gekennzeichnet, daß** der Fettalkohol die Struktur R-OH, worin R für einen linearen oder verzweigten, gesättigten oder ungesättigten Rest mit 8 bis 40 Kohlenstoffatomen und vorzugsweise 8 bis 30 Kohlenstoffatomen steht, aufweist.

12. Zusammensetzung nach Anspruch 10, **dadurch gekennzeichnet, daß** der Fettalkohol unter Cetylalkohol, Stearylalkohol und Mischungen davon ausgewählt ist.

13. Zusammensetzung nach einem der vorhergehenden Ansprüche, **dadurch gekennzeichnet, daß** der Fettalkohol in der Zusammensetzung in einer Menge zwischen 0,01 und 10 Gew.-%, vorzugsweise zwischen 0,1 und 5 Gew.-% und weiter bevorzugt zwischen 0,2 und 4 Ges.-%, bezogen auf das Gesamtgewicht der Zusammensetzung, vorliegt.

14. Zusammensetzung nach einem der vorhergehenden Ansprüche, **dadurch gekennzeichnet, daß** das Treibmittel unter gegebenenfalls halogenierten leichtflüchtigen Kohlenwasserstoffen, Kohlendioxid, Stickstoffmonoxid, Dimethylether (DME), Stickstoff, Druckluft und Mischungen davon ausgewählt ist.

15. Zusammensetzung nach Anspruch 14, **dadurch gekennzeichnet, daß** der leichtflüchtige Kohlenwasserstoff unter n-Butan, Propan, Isobutan, Pentan und Mischungen davon ausgewählt ist.

16. Zusammensetzung nach Anspruch 14, **dadurch gekennzeichnet, daß** es sich bei dem Treibmittel um Dimethylether handelt.

17. Zusammensetzung nach einem der vorhergehenden Ansprüche, **dadurch gekennzeichnet, daß** das Treibmittel in der Zusammensetzung in einer Menge zwischen 1 und 15 Gew.-%, vorzugsweise zwischen 2 und 10 Gew.-% und weiter bevorzugt zwischen 3 und 8 Gew.-%, bezogen auf das Gesamtgewicht der Zusammensetzung, vorliegt.

18. Zusammensetzung nach einem der vorhergehenden Ansprüche, **dadurch gekennzeichnet, daß** das kosmetisch unbedenkliche Medium aus Wasser oder einem oder mehreren kosmetisch unbedenklichen Lösungsmitteln oder Mischungen von Wasser und Lösungsmittel(n) besteht.

19. Zusammensetzung nach Anspruch 18, **dadurch gekennzeichnet, daß** das kosmetisch unbedenkliche Lösungsmittel unter Ethanol und Isopropanol ausgewählt ist.

20. Zusammensetzung nach Anspruch 19, **dadurch gekennzeichnet, daß** es sich bei dem kosmetisch unbedenklichen Lösungsmittel um Ethanol handelt.

21. Zusammensetzung nach einem der vorhergehenden Ansprüche, **dadurch gekennzeichnet, daß** sie außerdem mindestens ein unter Duftstoffen, Filtern, nichtionischen, anionischen, amphoteren oder zwitterionischen Tensiden, Konservierungsmitteln, Proteinen, Vitaminen, nichtionischen, anionischen, kationischen, amphoteren oder zwitterionischen Polymeren, Mineralölen, Pflanzenölen oder synthetischen Ölen, Mitteln gegen Schuppen, Mitteln gegen Haarausfall, Farbstoffen, Pigmenten, Reduktionsmitteln, Silikonen ohne Aminogruppen, Pflanzenwachsen und Ceramiden ausgewähltes Additiv enthält.

22. Aerosolvorrichtung, die eine Zusammensetzung gemäß einem der Ansprüche 1 bis 21 enthält.

23. Verwendung einer Zusammensetzung gemäß einem der Ansprüche 1 bis 21 zur kosmetischen Behandlung der Haare und der Kopfhaut.

24. Verfahren zur kosmetischen Behandlung der Haare, **dadurch gekennzeichnet, daß** man auf die Haare eine kosmetische Zusammensetzung gemäß einem der Ansprüche 1 bis 21 aufbringt.

25. Verfahren nach Anspruch 24, **dadurch gekennzeichnet, daß** das Aufbringen nach einer Haarwäsche erfolgt.
